# EUROPEAN PATENT APPLICATION

(11) **EP 4 450 067 A1**
(43) Date of publication of application: **23.10.2024**
(21) Application number: 24169162.5
(22) Date of filing: 09.04.2024
(51) Int. Cl.: A61K 9/16, A61K 9/20

(54) **PHARMACEUTICAL, DIETETIC, AND/OR FOOD FORMULATION BASED ON ADEMETIONINE AND PROCESS FOR MAKING SAID FORMULATION**

(30) Priority: 19.04.2023 IT 202300007692
(71) Applicant: Vifra S.r.L., 03013 Ferentino (FR) (IT)
(72) Inventor: Fortuna, Virgilio, Ferentino (IT); Cappucci, Francesco, Ferentino (IT); Mezzina, Cosmo, San Giuliano Milanese (IT); Saccoccio, Stefania, Patrica (IT)
(74) Representative: Lunati & Mazzoni S.r.L.

(57) **Abstract**

A pharmaceutical, dietetic, and/or food formulation based on granular, capsule, or tablet ademetionine is provided, comprising a granule base, wherein the granules include ademetionine or its salts in an amount between 85% and 95% by weight of the formulation, an anti-acid adjuvant in an amount between 0.5% and 3.5% by weight of the formulation, and vegetable fats in an amount between 5% and 11% by weight of the formulation.

## Description

This invention relates to a pharmaceutical, dietetic, and/or food formulation based on ademetionine and a process for making said formulation of the type specified in the preamble of the first claim.

In particular, this invention relates to a formulation that includes ademetionine, also known as SAMe or S-adenosyl methionine, stabilized and its making process, for the production of products in granular and/or capsule form and/or in tablet form. As known, ademetionine is a highly hygroscopic raw material that degrades quickly when coming into contact with the relative humidity of the environment. This characteristic makes it very difficult, if not impossible, to process it and mix it with other substances under standard environmental conditions.

Therefore, a significant drawback of the known technique is that making formulations including ademetionine salts is extremely difficult. Moreover, there are no known processes that allow for the making of granular or capsule formulations or tablets that effectively stabilize ademetionine without the addition of particular additive substances.

Indeed, for example, patent application EP-B-1971370 describes a stabilization process of ademetionine that includes the addition of inositol and/or its derivatives to stabilize the formulation including ademetionine. However, such patent application acknowledges that in the absence of such an additive, it is necessary to resort to particularly burdensome procedures involving strict control of humidity, impurities, and the making of coatings or coverings to protect the formulation, for example when in tablet form.

In this context, the technical task underlying this invention is to devise a pharmaceutical, dietetic, and/or food formulation based on ademetionine and a process for making said formulation capable of substantially overcoming at least part of the cited drawbacks.

Within this technical task, an important aim of the invention is to obtain a pharmaceutical, dietetic, and/or food formulation and process for making said formulation that allow defining more stable products, with better workability and improved palatability that can promote oral intake, given that one of the best absorption sites is the buccal mucosa.

Another important aim of the invention is to create a pharmaceutical, dietetic, and/or food formulation and process for making said formulation that is economical and involves simplified making phases.

The technical task and the specified aims are achieved by a pharmaceutical, dietetic, and/or food formulation based on ademetionine and process for making said formulation as claimed in the attached claim 1. Preferred technical solutions are highlighted in the dependent claims.

In this document, measurements, values, shapes, and geometric references (such as perpendicularity and parallelism), when associated with words like "approximately" or other similar terms such as "almost" or "substantially", are to be understood as allowing measurement errors or inaccuracies due to production and/or manufacturing errors and, above all, a slight divergence from the value, measurement, shape, or geometric reference to which it is associated. For example, such terms, if associated with a value, preferably indicate a divergence not exceeding 10% of the value itself.

Furthermore, when used, terms like "first", "second", "upper", "lower", "main", and "secondary" do not necessarily identify an order, a relationship priority, or relative position, but can simply be used to more clearly distinguish different components one from the other. Measurements and data reported in this text are to be considered, unless otherwise indicated, as conducted in International Standard Atmosphere ICAO (ISO 2533:1975).

The pharmaceutical, dietetic, and/or food formulation based on ademetionine object of this invention can be used for making semi-finished and/or finished products, whether they consist of granular or capsule configurations or tablets of the formulation itself.

Essentially, the term semi-finished refers to the formulation prepared to be subsequently mixed with other components or reprocessed for example with known processing methods.

The term "finished product", instead, refers to the formulation ready for compression, encapsulation, or packaging.

Therefore, the finished product is the product defined with the formulation according to the invention ready for use by any consumer, the semi-finished is defined by the formulation according to the invention ready to be subsequently processed and/or mixed with other components to create the finished product.

Finished products made with this formulation are preferably used for the treatment of depression, cognitive dysfunctions, senile dementia, liver problems, osteoarticular problems.

The formulation according to this invention preferably constitutes a semi-finished product. Moreover, the formulation according to the invention is preferably also at least part of a finished product. The formulation might also be the finished product itself.

The formulation preferably includes a plurality of components. In particular, the formulation preferably includes at least one main substance or raw material, a low-melting binder, and an anti-acid adjuvant.

Preferably, the main substance consists of ademetionine or its salts. Ademetionine, also known as SAMe or S-adenosyl methionine, is essentially a coenzyme involved in the transfer of methyl groups. As known, the molecule is synthesized from methionine adenosyltransferase from adenosine triphosphate (ATP) and methionine.

In general, ademetionine is a substance with a strongly acidic and unpleasant taste and highly hygroscopic that degrades quickly when coming into contact with the relative humidity of the environment.

The formulation includes, in detail, preferably SAMe disulfate p-toluenesulfonate. The low-melting binder is, instead, preferably made of vegetable fats.

Even more in detail, vegetable fats may include vegetable stearin or stearic acid. Vegetable stearin, or stearic acid, is a multicomponent substance of fatty acids, predominantly C16 - C18. It is a UVCB substance, identified by the CAS number 6701-03-5 (associated with the REACH Registration number). This CAS number is not associated with an INCI name: by convention, the INCI name of "Stearic Acid" is associated.

In general, stearin has essentially a melting point between 53 and 59°C and is particularly resistant and waterproof to water.

The anti-acid adjuvant is, preferably, made of magnesium hydroxide, or Mg(OH)₂. Magnesium hydroxide is an inorganic compound deriving, in nature, from brucite mineral and widely known in the current state of the art.

Therefore, preferably, the formulation includes a granule base. The granule base preferably includes ademetionine or its salts, magnesium hydroxide or its salts, and vegetable fats.

Ademetionine is preferably included in the formulation in an amount between 85% and 95% by weight of the formulation itself.

The anti-acid adjuvant is preferably included in the formulation in an amount between 0.5% and 3.5% by weight of the formulation itself.

Vegetable fats are preferably included in the formulation in an amount between 5% and 11% by weight of the formulation itself.

Even more in detail, the granular base can be essentially made of ademetionine or its salts, anti-acid adjuvant, and vegetable fats.

Moreover, the granules can also be formed by an internal core and an external coating. In this latter configuration, preferably, the internal core includes ademetionine or its salts and magnesium hydroxide or its salts, while the external coating include vegetable fats.

Ademetionine or its salts, magnesium hydroxide, and vegetable fats can therefore also be included, in this embodiment, in the previously described amounts.

Even more in detail, also in this case, the internal core of the granular base can be essentially made of ademetionine or its salts and anti-acid adjuvant, and the external coating can be essentially made of vegetable fats.

The formulation, as already said, can be part of a finished product. The finished product can, therefore, include the formulation and other additive substances.

In particular, preferably, the finished product includes the formulation in an amount between 37% and 77% by weight of the finished product.

This percentage, in particular, varies depending on the type of finished product it is intended to make from the semi-finished product constituted by the formulation itself.

For example, the finished product is in granular state, and the formulation is in an amount between 39% and 47% by weight of the finished product.

Preferably, the formulation is, even more appropriately, present in an amount between 41% and 45% by weight of the finished product. Even more appropriately, the formulation is present in an amount approximately equal to 43% by weight of the finished product.

In a capsule or tablet form of the finished product, instead, preferably the formulation is in an amount between 67% and 75% by weight of the finished product.

Preferably, the formulation is, even more appropriately, present in an amount between 69% and 73% by weight of the finished product. Even more appropriately, the formulation is present in an amount approximately equal to 71% by weight of the finished product.

As already said, the finished product includes, preferably, one or more additive substances in addition to the formulation, that is, the semi-finished product including the main raw material, anti-acid adjuvant, and low-melting binder.

In particular, preferably, the formulation can include, in any embodiment, silicon dioxide in an amount between 0.2% and 4% by weight of the formulation. Depending on the embodiment, moreover, other additives can be provided. For example, in the granular embodiment, preferably the formulation includes xylitol in an amount between 43% and 63% by weight of the formulation.

In the capsule or complex embodiment, preferably, the formulation may include acid calcium phosphate in an amount between 15% and 25% by weight of the formulation and cellulose with a low moisture content MCC112 in an amount between 20% and 30% by weight of the formulation.

Some specific examples of embodiments of finished products including the formulation according to the invention are described in the following tables.

**Table 1 - Orosoluble Granules**

| | **Orosoluble Granules [mg]** | **%** |
|---|---|---|
| **Ingredients** | **2000** | |
| **SAMe-Stearin-Magnesium Hydroxide Blend** | **854.70** | **42.74** |
| - SAMe | 769.23 | 38.46 |
| - Stearic Acid | 68.38 | 3.42 |
| - Magnesium Hydroxide | 17.09 | 0.86 |
| **Saccharin** | **2.40** | **0.12** |
| **Sucralose** | **4.80** | **0.24** |
| **Flavour** | **50.00** | **2.50** |
| **Silicon Dioxide** | **10.00** | **0.50** |
| **Xylitol** | **1078.10** | **53.91** |

**Table 2 - Oblong Tablet**

| | **Oblong Tablet [mg]** | **%** |
|---|---|---|
| **Ingredients** | **1200.00** | |
| **SAMe-Stearin-Magnesium Hydroxide Blend** | **854.70** | **71.23** |
| - SAMe | 769.23 | 64.10 |
| - Stearic Acid | 68.38 | 5.70 |
| - Magnesium Hydroxide | 17.09 | 1.43 |
| **Silicon Dioxide** | **40.00** | **3.33** |
| **Dibasic Calcium Phosphate** | **140.00** | **11.67** |
| **Disocell** | **20.00** | **1.67** |
| **Cellulose MCC112** | **135.30** | **11.28** |
| **Magnesium Salts of Fatty Acids** | **10.00** | **0.83** |

In particular, as can be seen from the tables, ademetionine, anti-acid adjuvants, and vegetable fats appear as pre-mixed. Additives are then preferably added after the mixture is made. Among the additives, an amount of non-pre-mixed ademetionine may also be included.

To obtain the above-described formulation, the invention includes a new process for making the formulation involving at least two distinct phases for mixing the ingredients.

In particular, the process according to the invention includes pre-heating a container from room temperature to a first predetermined temperature. The container is, for example, a steel bin-type container including a jacket for collecting the components to be processed.

For example, during the pre-heating phase, the container can be heated to temperatures around 50°C.

Moreover, the process includes a phase of mixing ademetionine or its salts, anti-acid adjuvant, and vegetable fats within the container so as to make a base mixture. Preferably, the mixing occurs through a rotating blade for a time necessary to bring the mixture to a second predetermined temperature.

Appropriately, the second predetermined temperature coincides at least with the melting point of the vegetable fats. For example, therefore, the second predetermined temperature can be above 50°C, particularly between 53°C and 59°C.

During mixing, the rotating blade, or impeller, can be operated at speeds between 50 rpm and 100 rpm, for example, 80 rpm. The mixing phase may also include crushing the components of the base mixture through a chopper acting at speeds between 650 rpm and 750 rpm, for example, 700 rpm.

The base mixture, preferably, includes the components of the formulation, that is preferably ademetionine or its salts in an amount between 85% and 95% by weight of the base mixture, anti-acid adjuvant in an amount between 0.5% and 3.5% by weight of the base mixture, and the vegetable fats in an amount between 5% and 11% by weight of the base mixture.

Even more in detail, the base mixture may appropriately include ademetionine or its salts in an amount approximately equal to 90% by weight of the base mixture, anti-acid adjuvant in an amount approximately equal to 2% by weight of the base mixture, and the vegetable fats in an amount approximately equal to 8% by weight of the base mixture.

The process then includes a kneading phase of the base mixture. The base mixture is preferably kneaded until homogenization by decreasing the temperature of the container so as to maintain the base mixture stably at a temperature close to the second predetermined temperature.

By "close," it is meant that the temperature remains within tolerances of ± 10°C relative to the second predetermined temperature.

Even during kneading, the base mixture is subjected to the action of the rotating blade and the chopper. To appropriately knead the base mixture, preferably, the rotating blade may assume speeds between 100 rpm and 200 rpm, for example, 150 rpm. The chopper may continue to crush the components of the mixture at identical or similar speeds to the mixing phase.

The process then includes a cooling phase down to room temperature. In this phase, the mixture in the container can be left to rest, or, preferably, it can be subjected to the action of the rotating blade at contained speeds. Preferably, the rotating blade has, in this phase, a speed lower than 50 rpm, for example, 20 rpm.

The process then includes a milling phase aimed at allowing the transformation of the base mixture into the granule base included in the formulation.

At the end of the milling phase, the formulation is in the state of a finished product or semi-finished product.

When the formulation is used to make a finished product with the addition of additive substances, appropriately, the process includes an addition phase. In the addition phase, preferably, the additive substances are added to the granular base so as to make the finished product. Among the additive substances, as already said, an amount of non-pre-mixed ademetionine may also be foreseen.

This phase may preferably, but not necessarily, occur in the same container.

In conclusion, the process includes an auxiliary mixing phase, within the container, of the formulation so as to make the finished product.

The finished product could be, for example, a product as described in tables 1-2.

This auxiliary mixing phase can occur according to different durations and modes. For example, the auxiliary mixing can occur in two distinct phases wherein the formulation undergoes rotation in opposite directions. These phases can have, for example, durations of approximately 40 minutes each.

In any case, the containers, rotating blades, choppers, any heating and cooling apparatuses, rotating containers for auxiliary mixing, are devices known to those skilled in the art and do not require particular measures except those required by the type of machinery in use.

For example, the process according to the invention, or part of it, can be made with a machine HSMG ROTOCUBE 120 marketed by IMA^{™}.

In short, the ROTOCUBE 120 machinery is a machine that allows, using the measures described for the process according to the invention, the processing of powders and mixtures of powders in order to obtain granulates with better technological characteristics of flowability, compressibility index, and other relevant characteristics for similar substances.

The aforementioned machine allows various types of processing: wet granulation, melt granulation, pellet formation.

These processes are carried out inside a closed container, at controlled temperature, wherein the powders undergo the processes of mixing, wetting, granulation, and low-temperature drying.

In particular, the drying process is carried out under vacuum conditions, with cooling and subsequent milling.

The process according to the invention essentially involves a melt granulation procedure.

The phases previously described for the implementation of the process for making the formulation are preferably implemented in the order in which they are described. However, alternatives dependent on the type of additive to be added to the granule base could also be foreseen.

Below are some relevant parameters that define the process phases performed with the aforementioned ROTOCUBE 120 machinery.
- Pre-heating Phase:
   - Heating Time (min): 20,
   - Initial Container Temperature (°C): 23.8,
   - Final Container Temperature (°C): 50,
- Mixing Phase:
   - Mixing Time (min): 5,
   - Rotating Blade Speed (rpm): 80,
   - Crusher or Chopper Speed (rpm): 700,
   - Initial Internal Temperature of the Base Mixture (°C): 39.1,
   - Final Internal Temperature of the Base Mixture (°C): 57.8,
   - Initial Container Jacket Temperature (°C): 77.5,
   - Final Container Jacket Temperature (°C): 82.6,
- Kneading Phase:
   - Kneading Time (min): 9,
   - Rotating Blade Speed (rpm): 150,
   - Crusher Speed (rpm): 700,
   - Initial Internal Temperature of the Base Mixture (°C): 58.3,
   - Final Internal Temperature of the Base Mixture (°C): 66.3,
   - Initial Container Jacket Temperature (°C): 79.5,
   - Final Container Jacket Temperature (°C): 72.9,
- Cooling Phase:
   - Cooling Time (min): 30,
   - Rotating Blade Speed (rpm): 20,
   - Initial Internal Temperature of the Base Mixture (°C): 64.8,
   - Final Internal Temperature of the Base Mixture (°C): 45.6,
   - Initial Container Jacket Temperature (°C): 72.9,
   - Final Container Jacket Temperature (°C): 24.6.

The pharmaceutical, dietetic, and/or food formulation based on ademetionine and the process for making said formulation according to the invention achieve significant advantages. Indeed, the finished products made with the formulation and the related process are effectively workable since the issues related to the hygroscopicity of ademetionine are solved by the method according to the invention. Moreover, the presence of the acid adjuvant in the formulation significantly increases user compliance, especially in terms of pleasantness or palatability, which is not affected by the common issues related to the use of ademetionine. However, the anti-acid adjuvant does not compromise the effectiveness of the formulation whether used as a semi-finished product for the making of subsequent finished products including additive substances, or in the case where the formulation itself is the finished product. Furthermore, the process can be carried out simply and economically, and therefore, the process and the consequent formulation turn out to be cost-effective, albeit in light of the mentioned advantages. In particular, the mentioned advantages extend to any finished product according to the invention, whether it consists of the formulation or includes the formulation and additive substances, especially regarding therapeutic uses for the treatment of depression, cognitive dysfunctions, senile dementia, hepatic issues, and osteoarticular issues. The invention is susceptible to alternatives falling within the scope of the inventive concept defined by the claims. Within this scope, all details can be replaced by equivalent elements, and the materials, shapes, and dimensions can be any.

## Claims

1. A pharmaceutical, dietetic, and/or food formulation based on ademetionine in granular, capsule, or tablet form comprising a granule base, wherein said granules include ademetionine or its salts in an amount between 85% and 95% by weight of the formulation, an anti-acid adjuvant in an amount between 0.5% and 3.5% by weight of the formulation, and vegetable fats in an amount between 5% and 11% by weight of the formulation.

2. The formulation according to claim 1, wherein said granule base is essentially made of ademetionine or its salts, said anti-acid adjuvant, and said vegetable fats.

3. The formulation according to any preceding claim, wherein said granules are formed by an internal core, comprising said ademetionine or its salts and said anti-acid adjuvant, and by an external coating comprising said vegetable fats.

4. The formulation according to any preceding claim, wherein said anti-acid adjuvant is made of magnesium hydroxide or its salts.

5. The formulation according to any preceding claim, wherein said vegetable fats are made of vegetable stearin or stearic acid.

6. The finished product including a formulation according to any preceding claim in an amount between 37% and 77% by weight of the finished product and additive substances.

7. The product according to claim 6, wherein said additive substances include silicon dioxide in an amount between 0.2% and 4% by weight of the product.

8. The product according to any of claims 6-7, in granular form wherein said formulation is present in an amount between 39% and 47% by weight of the formulation.

9. The product according to claim 8, wherein said additive substances include xylitol in an amount between 43% and 63% by weight of the product.

10. The product according to any of claims 6-7, in capsule or tablet form wherein said formulation is present in an amount between 67% and 77% by weight of the formulation.

11. The product according to claim 10, wherein said additive substances include acid calcium phosphate in an amount between 15% and 25% by weight of the product and microcrystalline cellulose in an amount between 20% and 30% by weight of the product.

12. The process for making a pharmaceutical, dietetic, and/or food formulation in granular, capsule, or tablet form according to any of claims 1-4, comprising:
- pre-heating a container from room temperature to a first predetermined temperature;
- mixing within said container said ademetionine or its salts, said anti-acid adjuvant, and said vegetable fats, so as to define a base mixture, using a rotating blade for a time necessary to bring said base mixture to a second predetermined temperature coinciding with the melting point of said vegetable fats;
- kneading said base mixture until homogenization by decreasing the temperature of said container so as to maintain said base mixture stably at a temperature close to said second predetermined temperature;
- cooling said container to room temperature;
- milling said base mixture so as to make said granule base.

13. The process according to the preceding claim, wherein said base mixture includes said ademetionine or its salts in an amount between 85% and 95% by weight of the base mixture, said anti-acid adjuvant in an amount between 0.5% and 3.5% by weight of the base mixture, and said vegetable fats in an amount between 5% and 11% by weight of the base mixture.

14. The process according to any of claims 12-13, further comprising:
- adding additive substances to said formulation in a second container;
- further mixing, in an auxiliary mixing phase, said formulation and said additive substances within said second container so as to make a finished product according to any of claims 6-11.
